# EUROPEAN PATENT SPECIFICATION

(11) **EP 1 357 936 B1**
(45) Date of publication and mention of the grant of the patent: **01.10.2008**
(21) Application number: 02707532.4
(22) Date of filing: 16.01.2002
(51) Int. Cl.: A61L 27/04, A61L 27/30, A61L 27/54, A61L 29/02, A61L 29/08, A61L 29/16, A61L 31/02, A61L 31/08, A61L 31/16

(54) **BIOCATALYTIC AND BIOMIMETIC GENERATION OF NITRIC OXIDE IN SITU AT SUBSTRATE/BLOOD INTERFACES**
BIOKATALYTISCHE UND BIOMIMETISCHE ERZEUGUNG VON STICKOXID IN SITU AN SUBSTRAT/BLUT-GRENZFLÄCHEN
GENERATION BIOCATALYTIQUE ET BIOMIMETIQUE IN SITU D'OXYDE NITRIQUE SUR DES INTERFACES SUBSTRAT/SANG

(30) Priority: 16.01.2001 US 262014 P
(43) Date of publication of application: 05.11.2003
(73) Proprietor: THE REGENTS OF THE UNIVERSITY OF MICHIGAN, Ann Arbor, Michigan 48109-1280 (US)
(72) Inventor: MEYERHOFF, Mark, E., Ann Arbor, MI 48103 (US); BATCHELOR, Melissa, M. University of Michigan, Ann Arbor, MI 48105 (US); OH, Bong, Kyun University of Michigan, Ann Arbor, MI 48105 (US)
(74) Representative: Steimle, Josef
(86) International application number: PCT/US2002/001687
(87) International publication number: WO 2002/056904

(56) References cited:
- WO-A-00/02501
- WO-A-00/11965
- WO-A-00/12112
- WO-A-00/27887
- WO-A-95/04078
- WO-A-99/09912
- US-A- 3 933 589
- US-A- 4 339 448
- US-A- 5 283 339
- US-A- 5 294 539
- US-A- 5 386 012
- US-A- 5 834 030
- US-A- 6 033 368
- US-A- 6 143 556
- DOEL J J ET AL: "Reduction of organic nitrites to nitric oxide catalyzed by xanthine oxidase: possible role in metabolism of nitrovasodilators." BIOCHEMICAL AND BIOPHYSICAL RESEARCH COMMUNICATIONS. 21 APR 2000, vol. 270, no. 3, 21 April 2000 (2000-04-21), pages 880-885, XP001205365 ISSN: 0006-291X
- FROST M C ET AL: "Polymers incorporating nitric oxide releasing/generating substances for improved biocompatibility of blood-contacting medical devices" BIOMATERIALS, ELSEVIER SCIENCE PUBLISHERS BV., BARKING, GB, vol. 26, no. 14, May 2005 (2005-05), pages 1685-1693, XP004670979 ISSN: 0142-9612
- CHANDRA, SULEKH ET AL: "Synthesis and spectral studies on copper(II) complexes of two twelve-membered and tetradentate macrocyclic ligands" INDIAN JOURNAL OF CHEMISTRY, SECTION A: INORGANIC, BIO-INORGANIC, PHYSICAL, THEORETICAL & ANALYTICAL CHEMISTRY, 37A(12), 1074-1078 CODEN: ICACEC; ISSN: 0376-4710, 1998, XP009048670

## Description

### Government Rights

This invention was made under contract awarded by the National Institutes of Health, Contract Number F-002881. The government has certain rights in the invention.

### Relationship to Other Application(s)

This application is a continuation-in-part of U.S. Serial No. 60/262,014 filed on January 16, 2001, and claims the benefit thereof.

### Background of the Invention

### FIELD OF THE INVENTION

This invention relates generally to biocompatible materials, such as polymers or metals, and more particularly, to biocompatible materials having blood interface surfaces that are capable of biocatalytic or biomimetic generation of nitric oxide *in situ* when contacted with endogenous nitrite/nitrate or nitrosothiols in blood.

### DESCRIPTION OF THE RELATED ART

Although medical devices such as extracorporeal circuits and hemodialysis tubes are widely used in clinical settings, the polymers typically used to fabricate such devices (PVC, polyurethane, silicone rubber, *etc.*) are still subject to platelet aggregation and adhesion onto the surface of these materials. Thus, patients are often given anti-clotting agents (*i.e*., heparin) in order to reduce thrombosis on the surface of these devices. Similarly, implanted devices made of stainless steel or other alloys, or even carbon, can cause thrombus formation when in direct contact with blood. There is, therefore, a need for materials that more closely simulate the antithrombogenic properties of the endothelial cells that line blood vessels in order to obviate the need to administer anticoagulants.

Nitric oxide (NO) is an important intra- and intercellular messenger molecule that plays an important physiological role in platelet anti-activation, vascular relaxation, neurotransmission, and immune response. It has been proposed that synthetic materials that release low levels of NO would, therefore, more closely simulate the natural activity of endothelial cells, and therefore, would have improved biocompatibility.

Several classes of NO-releasing materials are currently under investigation worldwide. These include NO donors (*i.e*., diazeniumdiolates, nitrosothiols) are relatively complicated to synthesize and require stringent storage conditions. Thus, there is a need for improved materials that are easier to fabricate and store.

Currently, NO generation is determined by water uptake (such as in the case of diazeniumdiolates) or the intensity of light (as with iron nitrosyls). However, blood already contains a host of species that are derived from, or are physiologically-generated *in vivo* that can be reduced to NO. These species include, nitrites, nitrates, and a host of nitrosothiols (*e.g*., nitrosoglutathione, nitroso albumin, *etc*.). This raises the possibility of recycling these species back to nitric oxide. There is, therefore, a need for materials that can reduce these species to nitric oxide locally at the substrate/blood interface.

It is an object of this invention to provide improved materials for biomedical applications that are capable of releasing NO from blood-contacting surfaces materials, so as to prevent platelet activation and adhesion onto these surfaces, thereby lowering thrombus formation and other complications associated with interactions between blood and foreign materials.

It is a further object of this invention to provide improved materials for biomedical applications that are relatively inexpensive to manufacture and that have improved biocompatibility.

It is still a further object of this invention to provide materials for biomedical applications that are capable of releasing NO from blood-contacting surfaces materials in response to nitrites/nitrates and nitrosothiols in the blood.

### Summary of the Invention

The foregoing and other objects are achieved by this invention which provides a novel approach for enhancing the biocompatibility of materials of the type suitable for implantation in a human or animal body and/or for prolonged contact with the body or blood. In accordance with a broad aspect of the invention, materials have been developed to have a catalytic surface that is capable of generating, at the catalytic surface/blood interface, physiologically significant amounts of NO when in contact with blood. A catalytic agent, having nitrite reductase and/or nitrite reductase-like activity, or a nitrosothiol reductase activity, is immobilized, adsorbed, adhered, or otherwise made available at a surface of the material.

The catalytic agent is a biomimetic catalytic agent. As used herein the term "biomimetic catalytic agent" refers to a species possessing nitrite reductase-like activity, or the ability to reduce nitrosothiols which converts endogenous or exogenous nitrite/nitrate or nitrosothiols to NO when in contact with blood.

The biomimetic catalytic agent is a metal ion ligand complex wherein the metal ion is capable of reducing one or more of nitrate, nitrate, nitrosothiols, and other blood species to nitric oxide.

The metal ion ligand complex is a Cu(II) complex. Neutral carrier type ligands that have high metal binding affinity, particularly for copper, and, preferably, planar square-type geometry that provides a minimum amount of steric hindarance to the approach of the electron source (*e.g*., ascorbate or NADH) to the center metal of the complex so that the copper ion can easily be reduced from Cu(II) to Cu(I), are suitable for the practice of the invention.

The biomimetic catalyst is a Cu(II) metal ion ligand complex is selected from the group consisting of dibenzo[e,k]-2,3,8,9-tetraphenyl-1,4,7,10-tetraaza-cyclododeca-1,3,7,9-tetraene; dibenzo[e,k]-2,3,8,9-tetramethyl-1,4,7,10-tetraaza-cyclododeca-1 ,3,7,9-tetraene; and dibenzo[e,k]-2,3,8,9-tetraethyl-1,4,7,10-tetraaza-cyclododeca-1,3,7,9-tetraene.

As used herein, the term "material," when referring to the material that is provided with the catalytic surface, may be any material, and preferably a material of a type that suitable for contact with the body and/or body fluids, particularly blood, of a living being, e.g., is physiologically acceptable, and non-toxic. In some embodiments, the material should be suitable for long-term contact, or in-dwelling uses. Broadly, such materials encompass polymers.

Many polymeric materials are suitable for the practice of the invention, and the following illustrative list of polymers that have been used for biomedical applications, is illustrative, and not intended to be limiting in any manner. Examples include synthetic polymers such as polyurethane, polydimethylsiloxane, ethylene vinyl acetate, nylons, polyacrylic, polymethyl methacrylate, polyamide, polycarbonate, polyester, polyethylene, polypropylene, polystyrene,polyvinyl chloride, polytetrafluoroethylene, and cellulose acetate.

In specific preferred embodiments, a material in accordance with the invention comprises a hydrophobic polymer substrate, such as poly(vinyl chloride), polyurethane, and silicone rubber, and a layer of a catalytic agent having nitrite reductase and/or nitrite reductase-like activity, or a nitrosothiol reductase activity attached to a surface of the hydrophobic polymer substrate. The attachment may be by adsorption, covalent bonding, and the like. The polymer substrate may, in some embodiments, include lipophilic salts of nitrite/nitrate or nitrosothiols within its matrix to create a reservoir of nitrite/nitrate or nitrosothiol that can continuously leak to the catalytic surface.

The NO-releasing polymer can be formed, cast, or otherwise shaped to comprise a monolithic device, such as implantable device such as drug depot or in-dwelling devices, such as catheters, or extracorporeal tubing sets (including kidney dialysis or open-heart surgery heart-lung machines). The polymer can also be applied as a film on another substrate that may be a polymer, or another surface, such as the surface of a metal device.

Suitable metals include, but are not limited to, stainless steel, nickel, titanium, aluminum, copper, gold, silver, platinum and combinations thereof. The metal material may comprise a medical devices, and the following types of devices, provided with a catalytic agent in accordance with the principles of the invention, are meant to be illustrative, but not limiting, examples: arterial stents, guide wires, catheters, bone anchors and screws, protective platings, hip and joint implants, spine appliances, electrical leads, biosensors and probes.

As stated above, the biomimetic catalytic agent is a metal ion ligand complex which is capable of reducing one or more of nitrate, nitrate, nitrosothiols, and other blood species to nitric oxide. The biomimetic catalytic agent is a Cu(II) metal ion ligand complex.

In certain preferred embodiments, an exogenous source of nitrite/nitrate or nitrosothiols is provided in the polymer matrix to create a reservoir of nitrite/nitrate or nitrosothiol that can continuously leak to the catalytic surface of the material. In these embodiments, the exogenous source, illustratively, lipophilic salts of nitrite/nitrate or nitrosothiols are dispersed within a polymer matrix material. In some embodiments, the polymeric material containing the exogenous source of nitrite/nitrate or nitrosothiol is applied to a catalytic surface as a coating. Illustrative source of nitrite/nitrate or nitrosothiol, include, without limitation, quaternary ammonium salts, such as tridodecylmethylammonium nitrite (TDMA⁺ + NO₂⁻/NO₃⁻); trimethyl phenyl ammonium; dimethyl dioctadecyl ammonium; etc. In addition to quaternary ammonium salts, quaternary phosphonium salts or quaternary arsonium salts may be used in the practice of the invention.

Methods of making the invention include swelling a polymer, such as polyvinyl chloride (PVC) or silicone, in the presence of an organic solvent containing an appropriate nitrite/nitrate salt to form a nitrite/nitrate salt-containing polymer.

In a other method embodiments, the biomimetic generation of NO can be further achieved by immobilizing metal-ion ligand complexes, on the surface of the material, or by dispersing these ligands within the material, which is a polymer. In some embodiments, additional lipophilic nitrite/nitrate salts, or nitrosothiols, are added to an underlying polymer matrix material or provided as a coating on the material, or as an additional layer.

### Brief Description of the Drawing

Comprehension of the invention is facilitated by reading the following detailed description, in conjunction with the annexed drawing, in which:
Fig. 1 is a schematic of illustration of NO generation in solution via nitrite reductase activity from the catalytic surface of in a polymer loaded with nitrite salt;
Fig. 2 is a graphical representation of the NO-release profile from nitrite ion-pair doped polymer films having immobilized XO on the surface in the presence of sheep blood;
Fig. 3 is a schematic representation of NO generation from a polymer matrix that has been loaded with a nitrate salt and a Cu(II) ligand complex in accordance with the invention;
Fig. 4 is a schematic representation of a material, in accordance with the invention, wherein a Cu(II) ligand complex is covalently tethered to the surface;
Fig. 5 is a is a graphical representation of the surface generation of NO from a Cu(II) ligand complex-containing polymer film in a bulk solution containing nitrite and ascorbate;
Fig. 6 shows three examples of illustrative metal ligand complexes; and
Fig. 7 is a graphical representation of NO generation from a nitrite ion pair/Cu(II) complex, specifically the complex designated L2 on Fig. 6.

### Detailed Description

In one method embodiment for making an improved NO-releasing polymer, the desired polymer may be swelled-in an organic solution containing the lipophilic nitrite/nitrate salt. In other embodiments, the salt can be added during the processing stage when the desired end product is molded or cast from the native polymer material. In still other embodiments, the surface of the polymer material that will be exposed to blood, for example, the outside surface of a catheter or the inner surface of tubing of the type used in extracorporeal circuits, may be coated, either by dip-coating or by another method with a biomimetic catalyst capable of reducing nitrate to NO or nitrite to NO, or nitrosothiols to NO. The biomimetic catalysts can also be covalently tethered to the surface of the material.

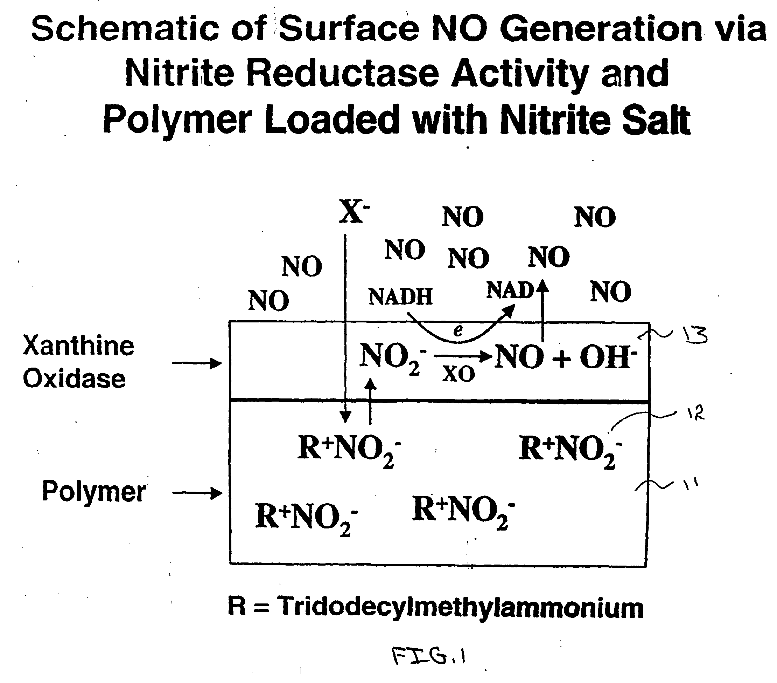

In a specific illustration (not an example of the invention), mammalian xanthine oxidase (XO) is used as the surface catalyst for nitrite reduction to NO. In the presence of nicotinamide adenine dinucleotide (NADH), or other reducing equivalents in blood, the surface catalyst will generate NO as the nitrite ions leak from within the material into this surface layer via exchange for chloride and bicarbonate within the blood. A schematic representation is illustrated in Fig. 1. Referring to Fig. 1, a polymer matrix 11 that has been loaded with a lipophilic nitrite/nitrate salt of tridodecylmethylammonium 12 (R⁺NO2⁻) that provides a source of nitrite ions (NO₂⁻). A coating 13 of xanthine oxidase 13 (XO).

Preliminary feasibility studies have been carried out to demonstrate the basic concept of this invention. Using xanthine oxidase as a model enzyme for nitrite reductase activity. PVC polymer films were doped with TDMA⁺ NO₂⁻ and then coated with a layer of immobilized XO.

Illustratively, the PVC polymeric film, or membrane, was prepared by a cocktail solution casting method as described, for example, in Mathison, et al., Anal. Chem., Vol. 71, pages 4614-4621 (1999) or any of the patents referenced herein. The cocktail solution was prepared by dissolving the appropriate amounts of membrane components (polymer, plasticizers and, in some cases, an ion-exchanger) into a solvent, illustratively tetrahydrofuran (THF). The membranes were cast in a mold to a final thickness of about 150 µm.

The polymer film was then coated with immobilized XO prepared by crosslinking XO with bovine serum albumin (BSA) in the presence of glutaraldehyde. The cross-linked product forms a hydrogel that is dip-coated on the PVC polymer substrate.

An electrochemical sensor was used to probe the surface concentrations of NO generated when the coated film were placed into a buffered solution containing NADH at physiological pH. Significant levels of NO were generated at the surface of the film under these conditions. The generation of NO near the surface of the polymer film continued for several hours as the nitrite in the film was exchanged for anions in the buffer phase.

In this particular study, the electrochemical NO sensor used was similar in style to conventional Clark type oxygen sensor. Glass coated Platinum (Pt) wire served as the anode and Ag/AgCl wire (0.25 mm dia.) was used as the cathode. The internal filling solution was composed of 0.3 mM HCl and 30 mM NaCl, pH 3.5. An outer gas permeable membrane (Goretex, polytetrafluoroethylene with 50% porosity and 0.2 µm pore size) was placed between the internal filling solution and sample solution. Amperometric NO measurements were performed using an electrochemical analyzer.

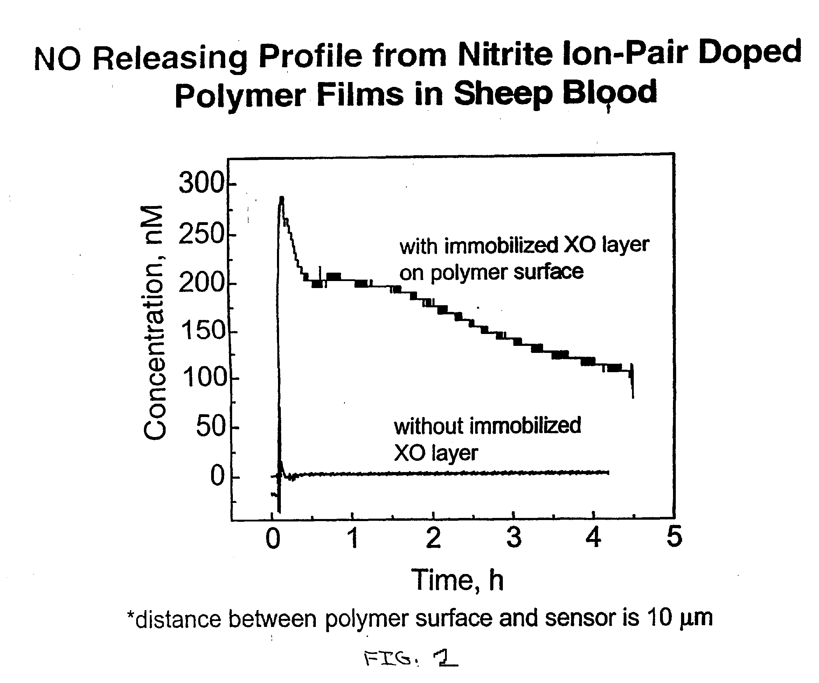

Fig. 2 graphically illustrates that, when a similar film coated with XO was exposed to whole sheep blood, without adding any reducing equivalents in the form of NADH, measurable levels of NO were generated at the surface of the film as detected by the aforementioned electrochemical NO sensor. This data suggests that there is adequate endogenous reducing equivalent species in blood to serve as the source of electrons for the biocatalytic reaction at the surface of a polymer prepared as described herein.

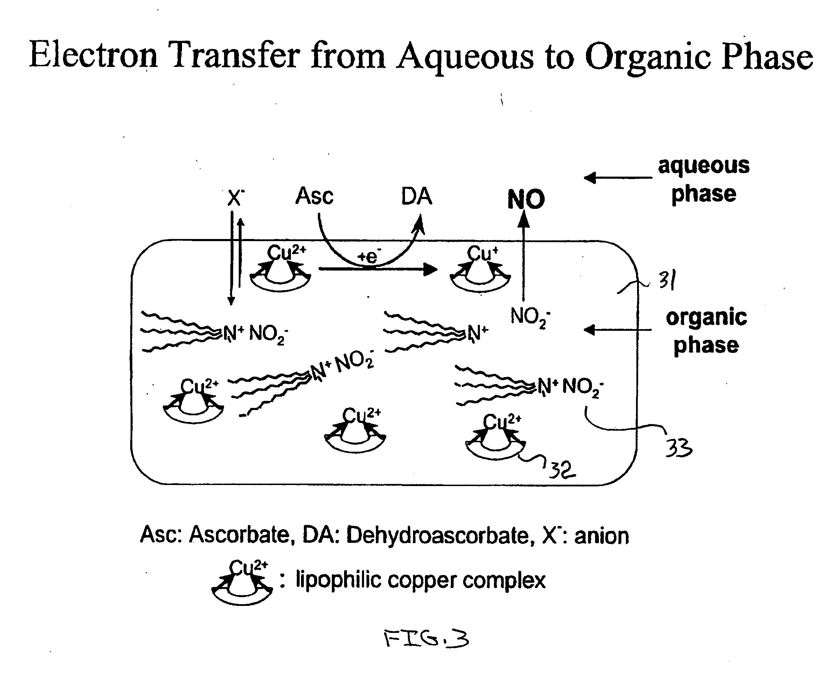

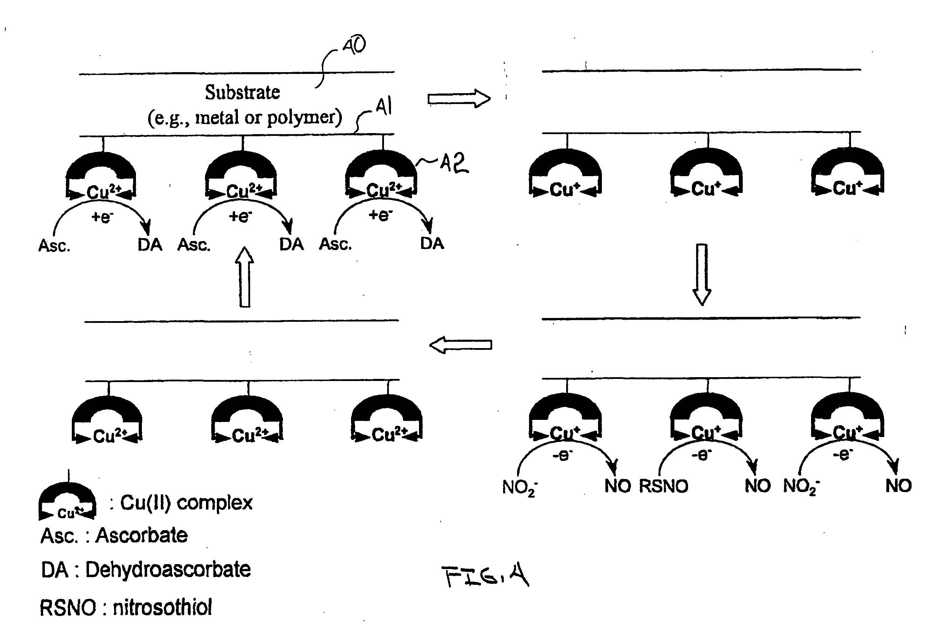

In an illustrative embodiment, biomimetic catalysts, such as Cu(II)-ligand complexes, for example, dibenzo[e,k]-2,3,8,9-tetraphenyl-1,4,7,10-tetraazacyclododeca-1,3,7,9-tetraene, were either incorporated in or tethered to a polymer or other material surface, such as a metal. Examples of this embodiment is shown in Figs. 3 and 4.

Fig. 3 is a schematic representation of a polymer matrix 31, illustratively PVC, that has been loaded with a lipophilic Cu(II) ligand complex 32 as well as a lipophilic nitrite/nitrate salt of tridodecylmethylammonium 33 (N⁺NO2⁻) that provides a source of nitrite ions (NO₂⁻) in the polymer. When the polymer is exposed to an aqueous solution containing ascorbate (ASC) or ascorbic acid, the ascorbic acid reduces the Cu(II) in the ligand complex to Cu(I). The Cu(I) in turn reduces nitrites in the film to NO.

Fig. 4 is a schematic representation of a material 40 that has a catalytic surface 41 created by tethering a Cu(II) ligand complex 42 to the surface. When the catalytic surface is exposed to an aqueous solution, which may be blood, containing ascorbic acid, the ascorbic acid reduces Cu(II) in the ligand to Cu(I). The Cu(I) returns to Cu(II) thereby converting nitrites and nitrosothiol (RSNO), for example, in the solution to NO.

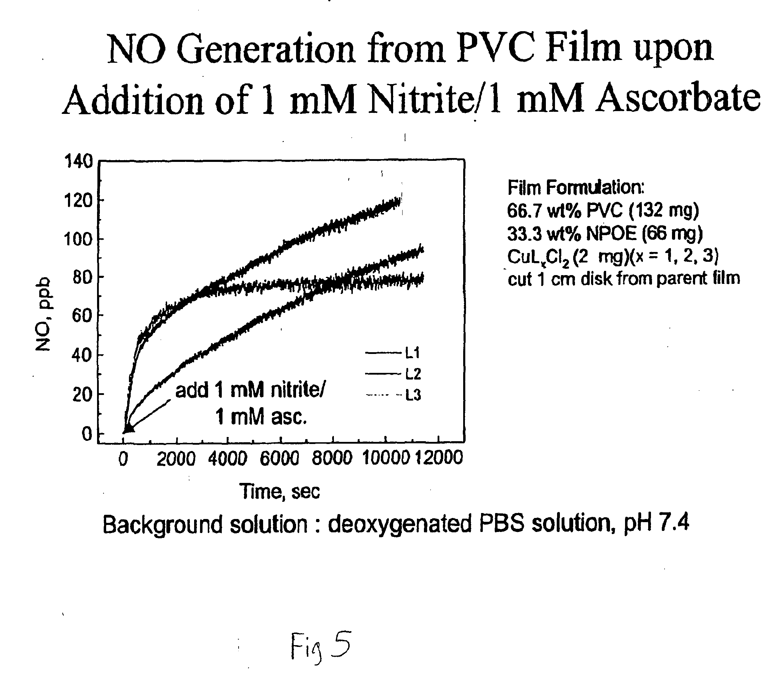

Fig. 5 is a graphical representation of the surface generation of NO from.a Cu(II) ligand complex-containing polymer film in a bulk solution containing nitrite and ascorbate. The data is plotted as NO concentration in parts per billion (ppb) as a function of time in seconds.

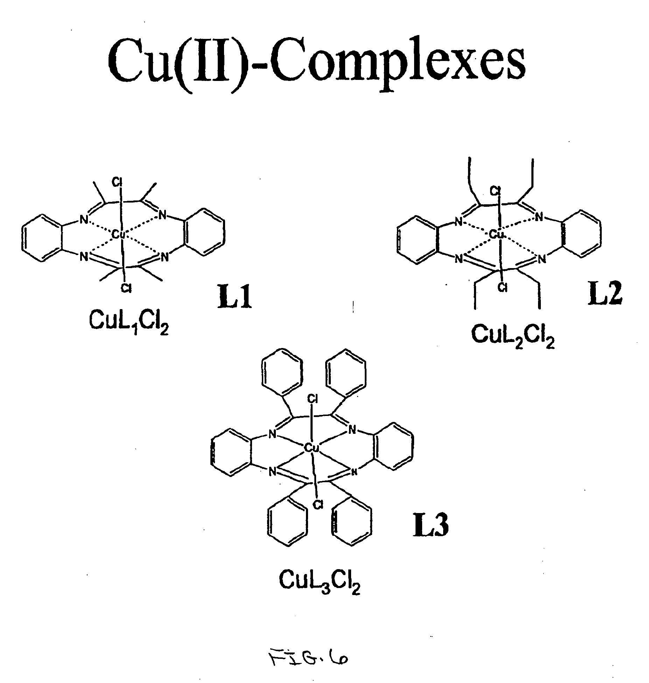

Three films having the following formulation were prepared in accordance with the method set forth above: 66.7 wt% PVC polymer (132 mg); 33.3 wt% plasticizer, illustratively nitrophenyloctyl ether (NPOE; 66 mg), and a Cu(II) ligand complex, CuLₓCl2 (2 mg), Lₓ being ligands, L1-L3 as shown on Fig. 6. The illustrative metal ligand complexes, specifically Cu(II) ligand complexes, shown on Fig. 6 are dibenzo[e,k]-2,3,8,9-tetraphenyl-1,4,7,10-tetraaza-cyclododeca-1,3,7,9-tetraene labeled L1; dibenzo[e,k]-2,3,8,9-tetramethyl-1,4,7,10-tetraaza-cyclododeca-1,3,7,9-tetraene labeled L2; and labeled L3 .

Although these complexes are shown as chloride salts, it is to be understood that other counterions are appropriate. Other metal ions were evaluated for activity, *i.e.,* ability to mediate the reduction of nitrite to NO by ascorbate, including Co(II), Ni(II), Zn(II) Mn(II), AI(II), and Fe(III). Of these ions, only Fe(III) yielded a detectable level of NO, but this was far less than that observe with Cu(II) under identical conditions. Other metals, such as V(III), Cr(III), and Ti(III) have also been suggested as being capable of reducing nitrite to NO. However, unlike Cu(II) (or Fe), these metals are not present in appreciable levels *in vivo,* either within physiological fluids, or within specialized cellular vesicles. Therefore, Cu(II) is presently the preferred metal ion for the practice of the invention.

Referring back to Fig. 5, traces 61, 62, and 63 being ligands L1-L3, respectively. In this particular experiment, the bulk solution was deoxygenated phosphate buffered saline (PBS) having a pH of 7.4. At time t=0, 1 mM nitrite and 1 nM ascorbate were added to the PBS solution and NO generation was measured with a chemiluminescense detector. The results demonstrate that films in accordance with the present invention are capable of NO generation at the interface when the nitrites and ascorbates are in the bulk solution, such as would occur when the films were placed in contact with blood in an *in vivo* situation.

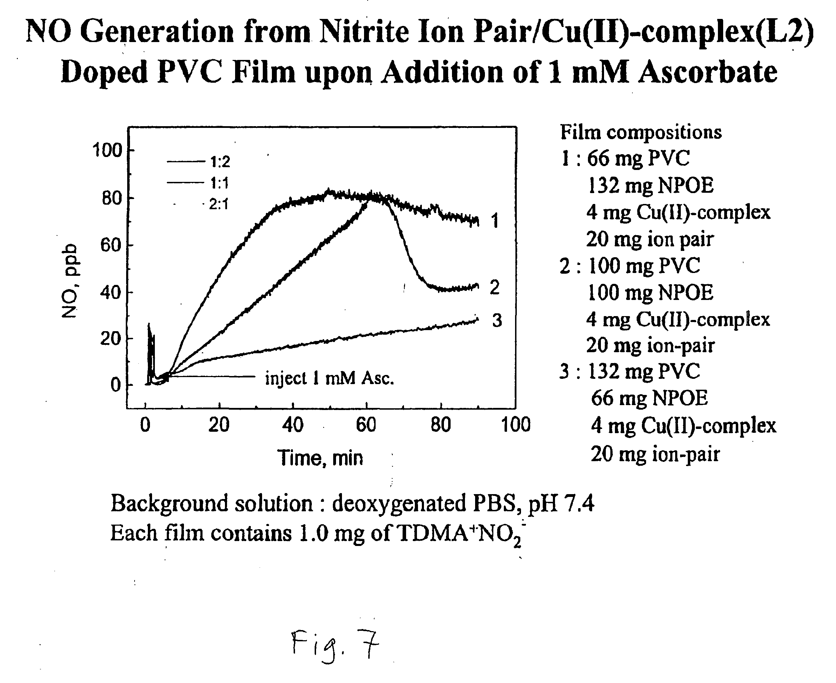

Fig. 7 is a graphical representation of NO generation from a nitrite ion pair/Cu(II) complex, specifically the complex designated L2 on Fig. 5A, doped into a polymer film. The data is plotted as NO concentration in parts per billion (ppb) as a function of time in minutes following introduction of 1mM ascorbate into a deoxygenated PBS solution having pH 7.4.

The polymeric film compositions used in this experiment are as follows:
Film 1:
   66 mg PVC; 132 mg NPOE; 4 mg Cu(II) complex; and 20 mg ion pair, orTDMA⁺NO₂⁻ .
Film 2:
   100 mg PVC, 100 mg NPOE, 4 mg Cu(II) complex; and 20 mg ion pair
Film 3:
   132 mg PVC; 66 mg NPOE; 4 mg Cu(II) complex; and 20 mg ion pair

These results show generation of NO by the polymer film that is particularly good for the highly plasticized embodiments.

The major advantage of this technology over the previous methods for generating
NO locally at the surface of polymers or other materials is the potential simplicity of simply dip-coating the material with a biomimetic catalytic layer. The catalytic layer may have a single catalyst or mixture of reductase activities. It can be a metal ion ligand complex that mimics the enzyme function. Even in those embodiments where added TDMA⁺ NO₂⁻/NO₃⁻ or some other nitrite/nitrate salt, or a nitrosothiol, such as nitroso cysteine, is required, or desired, within the polymer, the stability of such species is likely to far exceed the stability of diazeniumdiolates and other NO donors used to date.

In a clinical situation, it should be noted that, even if the amount of reducing equivalent species in the blood were to vary from test subject to test subject, it is possible to add reducing equivalents of an alternate electron donor to the blood, illustratively in the form of ascorbic acid, by administering low doses Vitamin C to the patient. This ensures the presence of adequate levels of reducing equivalents.

Although the invention has been described in terms of specific embodiments and applications, persons skilled in the art can, in light of this teaching, generate additional embodiments without exceeding the scope or departing from the spirit of the invention described herein. Accordingly, it is to be understood that the drawing and description in this disclosure are proffered to facilitate comprehension of the invention, and should not be construed to limit the scope thereof.

## Claims

1. A material comprising a) a polymer; and b) a Cu(II) metal ion ligand complex immobilized or available at a surface of said material, and having nitrite reductase or nitrosothiol reductase activity which converts nitrite/nitrate or nitrosothiols to nitric oxide when on contact with blood;
wherein the ligand is selected from the group consisting of: dibenzo [e,k]-2,3,8,9-tetraphenyl-1,4,7,10-tetraazacyclododeca-1,3,7,9-tetraene, dibenzo [e,k]-2,3,8,9-tetramethyl-1,4,7,10-tetraazacyclododeca-1,3,7,9-tetraene; and dibenzo [e,k]-2,3,8,9-tetraethyl-1,4,7,10-tetraazacyclododeca-1,3,7,9-tetraene

2. The material of claim 1, wherein the polymer is selected from the group of poly (vinyl chloride), polyurethane, and silicone rubber.

3. The material of claim 1, wherein the polymer further includes lipophilic salts of nitrite/nitrate or nitrosothiols within the polymer matrix to create a reservoir of nitrite/nitrate or nitrosothiol that can continuously leak to the catalytic surface.

4. The material of claim 3, wherein the lipophilic salt of nitite/nitrate is tridodecylmethylammonium nitrite (TDMA⁺ NO₂⁻/NO₃⁻).

5. A medical device comprising the material of claim 1.

6. The device of claim 6, wherein said device comprises a metal coated with the material of claim 1.

7. The medical device of claim 6, wherein the medical device is selected from the group consisting of arterial stents, guide wires, catheters, bone anchors and screws, protective platings, hip and joint implants, spine appliances, electrical leads, biosensors and probes.

## Patentansprüche

1. Material aus a) einem Polymer und b) einem Cu(II)-Metallionenligandenkomplex, der auf einer Oberfläche des Materials immobilisiert oder verfügbar ist und eine Nitritreduktase- oder Nitrosothiolreduktase-Aktivität aufweist, die Nitrit/Nitrat oder Nitrosothiole bei Kontakt mit Blut in Stickstoffoxid umwandelt;
wobei der Ligand aus der Gruppe bestehend aus Dibenzo[e,k]-2,3,8,9-tetraphenyl-1,4,7,10-tetraazacyclododeca-1,3,7,9-tetraen, Dibenzo[e,k]-2,3,8,9-tetramethyl-1,4,7,10-tetraazacyclododeca-1,3,7,9-tetraen und Dibenzo[e,k]-2,3,8,9-tetraethyl-1,4,7,10-tetraazacyclododeca-1,3,7,9-tetraen ausgewählt ist.

2. Material nach Anspruch 1, bei dem das Polymer aus der Gruppe bestehend aus Poly(vinylchlorid), Polyurethan und Siliziumkautschuk ausgewählt ist.

3. Material nach Anspruch 1, bei dem das Polymer weiterhin lipophile Salze von Nitrit/Nitrat oder Nitrosothiolen innerhalb der Polymermatrix enthält, um ein Reservoir von Nitrit/Nitrat oder Nitrosothiol zu erzeugen, das kontinuierlich in die katalytische Oberfläche sickern kann.

4. Material nach Anspruch 3, bei dem das lipophile Salz von Nitrit/Nitrat Tridodecylmethylammoniumnitrit (TDMA⁺NO₂⁻/NO₃⁻) ist.

5. Medizinische Vorrichtung, die das Material nach Anspruch 1 umfasst.

6. Vorrichtung nach Anspruch 5, wobei die Vorrichtung ein mit dem Material nach Anspruch 1 beschichtetes Metall umfasst.

7. Medizinische Vorrichtung nach Anspruch 6, das aus der Gruppe bestehend aus arteriellen Stents, Führungsdrähten, Katethern, Knochenankern und -schrauben, Schutzplatten, Hüft- und Gelenkimplantaten, Wirbelsäulengeräten, Elektroden, Biosensoren und Sonden ausgewählt ist.

## Revendications

1. Matériau comprenant a) un polymère et b) un complexe de Cu(II) métal / ligand ionique immobilisé ou disponible sur une surface dudit matériau, et possédant une activité de nitrite réductase ou de nitrosothiol réductase qui convertit les nitrites/nitrates ou les nitrosothiols en oxyde nitrique lorsqu'il est mis en contact avec le sang, **caractérisé en ce que** le ligand est choisi dans le groupe constitué par le dibenzo[e,k]-2,3,8,9-tétraphényl-1,4,7,10-tétraazacyclododéca-1,3,7,9-tétraène, le dibenzo[e,k]-2,3,8,9-tétraméthyl-1,4,7,10-tétraazacyclododéca-1,3,7,9-tétraène et le dibenzo[e,k]-2,3,8,9-tétraéthyl-1,4,7,10-tétraazacyclododéca-1,3,7,9-tétraène.

2. Matériau selon la revendication 1, dans lequel le polymère est choisi dans le groupe comprenant le polychlorure de vinyle, le polyuréthane et le caoutchouc silicone.

3. Matériau selon la revendication 1, dans lequel le polymère comprend en plus des sels nitrites/nitrates ou nitrosothiols lipophiles à l'intérieur de la matrice polymère afin de créer un réservoir de nitrite/nitrate ou de nitrosothiol capable de fuir en permanence vers la surface catalytique.

4. Matériau selon la revendication 3, dans lequel le sel nitrite/nitrate lipophile est le nitrite de tridodécylméthylammonium (TDMA⁺ NO₂⁻/NO₃⁻).

5. Dispositif médical comprenant le matériau selon la revendication 1.

6. Dispositif selon la revendication 6, dans lequel ledit dispositif comprend un métal revêtu du matériau selon la revendication 1.

7. Dispositif médical selon la revendication 6, dans lequel le dispositif médical est choisi dans le groupe constitué par des endoprothèses artérielles, des fils de guidage, des cathéters, des fixations et vis à os, des placages protecteurs, des prothèses de hanche et d'articulations, des appareils vertébraux, des câbles électriques, des biocapteurs et des sondes.
